# EUROPÄISCHE PATENTSCHRIFT

(11) **EP 0 341 593 B1**
(45) Veröffentlichungstag und Bekanntmachung des Hinweises auf die Patenterteilung: **21.12.1994**
(21) Anmeldenummer: 89108118.4
(22) Anmeldetag: 05.05.1989
(51) Int. Cl.: C08G 65/32, B01F 17/00

(54) **Modifizierte Blockpolymerisate, ihre Herstellung und Verwendung**
Modified block polymers, their preparation and use
Polymères séquencés modifiés, leur préparation et application

(30) Priorität: 11.05.1988 DE 3816126
(43) Veröffentlichungstag der Anmeldung: 15.11.1989
(73) Patentinhaber: HOECHST AKTIENGESELLSCHAFT, 65926 Frankfurt am Main (DE)
(72) Erfinder: Uhrig, Heinz, D-6374 Steinbach/Taunus (DE); Weide, Joachim, D-6233 Kelkheim (Taunus) (DE)

(56) Entgegenhaltungen:
- WO-A-86/00081
- DE-A- 2 260 969
- DE-A- 2 849 329
- US-A- 3 894 070
- US-A- 4 200 585
- PATENT ABSTRACTS OF JAPAN, Band 8, Nr. 273 (G-256), 13. Dezember 1984;& JP-A-59 145 293 (KAO SEKKEN K.K.) 02-08-1984

## Beschreibung

Die Erfindung betrifft das Gebiet oberflächenaktiver Substanzen, die beispielsweise als umweltfreundliche Dispergier- und Präparationsmittel für die Erzeugung von Farbmitteldispersionen, als Emulgiermittel für die Herstellung von Fett- und Mineralölemulsionen, als Kupplungshilfsmittel in der Synthese von Azopigmenten sowie als Färbereihilfsmittel, Netz- und Egalisiermittel eingesetzt werden können.

Bei der Herstellung von Präparationen von Farbmitteln, insbesondere von Pigmenten für die Anwendung in wäßrigen und organischen Medien werden häufig nicht-ionogene, anionaktive und auch kationaktive Tenside verwendet. Durch die oberflächenaktiven Eigenschaften solcher Tenside werden die coloristischen Eigenschaften in den verschiedenen Anwendungsmedien auf dem Drucksektor wesentlich beeinflußt. Auch bei der Herstellung von Azopigmenten und löslichen Azofarbstoffen werden üblicherweise zum besseren Ablauf der Kupplungsreaktion Tenside benutzt.

Aus der deutschen Auslegeschrift 10 81 226 sind bereits oberflächenaktive polymere Verbindungen bekannt, die durch Oxpropylierung und Oxethylierung von aliphatischen primären Diaminen erhältlich sind und welche als Reinigungsmittel bzw. Dispergiermittel für Kalkseifen verwendet werden.

In den deutschen Auslegeschriften 21 56 603 und 22 36 906 werden wäßrige, flockungsstabile Pigmentdispersionen beschrieben, die unter Zuhilfenahme von Blockpolymerisaten aus oxalkylierten aliphatischen, aromatischen oder cycloaliphatischen Diaminen zubereitet sind.

Die deutsche Offenlegungsschrift 22 60 969 erwähnt Monocarbonsäureester von Diaminoxethylaten und ihre Eignung als Arzneimittel.

Weiterhin werden in der japanischen Patentbekanntmachung Sho-59-071486 Blockpolymerisate von Ethylendiaminderivaten mit einem Alkylenoxid als Hilfsmittel beim Klotzen von Dispersionsfarbstoffen auf hydrophoben Synthesefasern erläutert. Schließlich sind aus der japanischen Patentbekanntmachung Sho-60-024229 styrolmodifizierte, teils mit Schwefelsäure sulfatierte Blockpolymerisate von oxalkylierten Alkylendiaminen für den Einsatz als Egalisiermittel beim Färben von synthetischen Materialien bekannt.

Erfindungsgemäß wurden nun strukturell modifizierte Blockpolymerisate ähnlichen Typs gefunden, die im Falle ihres Einsatzes auf den eingangs genannten Anwendungsgebieten gegenüber den Verbindungen vom Stand der Technik vielseitig verbesserte oberflächenaktive Eigenschaften aufweisen.

Gegenstand der vorliegenden Erfindung sind neue Verbindungen mit der Struktur eines durch mehrfache, gegebenenfalls unterschiedliche Veresterung modifizierten Blockpolymerisates (I), formelmäßig aufgebaut aus
a) 1 bis 10, vorzugsweise bis zu 5, trivalenten oder tetravalenten Blockoxalkylat-Einheiten auf Basis von Diaminen oder Polyaminen (Aminoxalkylate) mit der allgemeinen Formel (Ia) 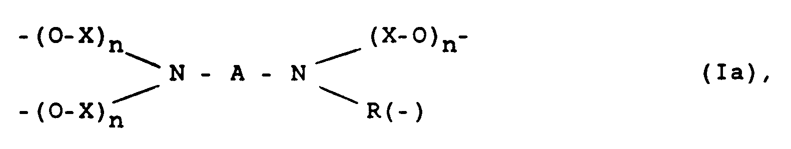
b) monovalenten, durch die allgemeine Formel (Ib)

   -Z (Ib)

   symbolisierten Säureresten,
und im Falle des Vorhandenseins von zwei oder mehreren Formeleinheiten (Ia) zusätzlich aus
c) divalenten Gruppen der allgemeinen Formel (Ic)

   - CO - B - CO - (Ic),

wobei jede der gezeigten freien Valenzen in den Formeleinheiten (Ia) so definiert ist, daß sie unabhängig voneinander mit jeweils einer Formeleinheit (Ib) oder einer Valenz der Formeleinheit (Ic) direkt verbunden ist, und wobei in den Formeleinheiten (Ia) bis (Ic)
- A: für einen geradkettigen, verzweigten oder cyclischen aliphatischen Rest, einen aromatischen oder araliphatischen Rest mit jeweils 1 bis 300 C-Atomen, vorzugsweise für C₂-C₁₂-Alkylen, insbesondere C₂-C₆-Alkylen, oder C₆-C₁₂-Arylen, C₇-C₁₄-Alkyl-arylen, C₅-C₇-Cyclo-alkylen oder C₂-C₂₄-Alkylen. das ein- oder mehrfach durch eine Gruppe der Formel NR′, worin R′ Wasserstoff, C₁-C₄-Alkyl oder eine Gruppe der Formel -(X-O)ₙ- mit einer wie oben definierten freien Valenz ist, unterbrochen ist, oder für eine Kombination der aufgeführten divalenten Gruppen,
- X: für gleiche oder verschiedene Gruppen der Formeln -CH₂CH₂- und -CH₂CH(CH₃)- , wobei die letztere Formel hier und im folgenden auch die Formel -CH(CH₃)CH₂-einschließen soll,
- n: für gleiche oder verschiedene Zahlen von 1 bis 100, insbesondere 1 bis 20,
- R: für eine C₁-C₃-Alkylgruppe oder eine divalente Gruppe der Formel -(X-O)ₙ- mit einer wie oben definierten, durch (-) angedeuteten freien Valenz,
- B: für einen geradkettigen, verzweigten oder cyclischen aliphatischen Rest, einen aromatischen oder araliphatischen Rest mit jeweils 1 bis 60 C-Atomen, vorzugsweise für geradkettiges C₁-C₈-Alkylen, einen Alkylenrest einer Dicarbonsäure auf Basis dimerisierter C₁₁-C₂₄-Fettsäuren, Cyclohexylen, C₆-C₁₂-Arylen oder eine Gruppe der Formel -CH=CH- oder -CH₂CH(SO₃M)-, worin M für ein Kation steht und die letzte Formel hier und im folgenden auch die Formel -CH(SO₃M)CH₂- einschließen soll, und
- Z: für gleiche oder verschiedene Reste Z¹ bis Z⁶, worin
- Z¹: Wasserstoff,
- Z²: einen Acrylrest der Formel R¹-CO-, in der R¹ ein geradkettiger, gesättigter oder ungesättigter C₇-C₂₁-aliphatischer KW-Rest, der nich durch ein oder zwei Hydroxygruppen oder durch einen C₆-C₁₂-Aryl- oder C₆-C₁₂-Hydroxyarylrest substituiert sein kann, vorzugsweise ein Alkylrest einer Fettsäure mit 12 bis 18 C Atomen,
- Z³: einen Acrylrest der Formel R²-CO-, in der R² einen geradkettigen, verzweigten oder cyclischen, gesättigten oder ungesättigten aliphatischen KW-Rest mit 1 bis 6 C-Atomen, der noch durch C₆-C₁₂-Aryl substituiert sein kann, oder einen Phenyl- oder Naphthylrest oder einen ein- bis dreifach substituierten Phenyl- oder Naphthylrest, wobei die Substituenten aus der Reihe C₁-C₁₂-Alkyl, C₁-C₁₂-Alkoxy, Sulfo, Hydroxy, Amino, C₁-C₁₂-Alkylamino, Di-(C₁-C₁₂-alkyl)amino, C₂-C₅-Acylamido, Acetoacetamido, Aminocarbonyl, C₁-C₄-Alkylamino-carbonyl und Di-(C₁-C₄-alkyl)-aminocarbonyl ausgewählt sind,
- Z⁴: einen Acylrest eine monofunktionellen Harzsäure vom Kolophoniumtyp,
- Z⁵: eine Gruppe der Formel -SO₃M, in der M ein Kation bedeutet, oder einen Acylrest der Formel -CO-B-COOM, in der B die definierte Bedeutung hat und M für ein Kation steht, und
- Z⁶: einen Acylrest der Formel -CO-B-COO- (X-O-)ₘ-Y-R³, in der B und X die genannten Bedeutungen haben, Y eine direkte Bindung oder eine Gruppe der Formel CO bedeutet, m im Falle Y = CO seine Zahl von 1 bis 150, vorzugsweise 1 bis 50, und im Falle Y = direkte Bindung eine Zahl von 0 bis 150, vorzugsweise 0 bis 50, und R³ ein gesättigter oder ungesättigter aliphatischer, aromatischer oder araliphatischer C₃-C₂₄-Kohlenwasserstoffrest, vorzugsweise Y-R³ ein Alkylrest einer Fettsäure mit 8 bis 20 C-Atomen, ein C₈-C₂₀-Alkylrest eines Fettalkohols, ein Acylrest einer monofunktionellen Harzsäure, Phenyl, Naphthyl, C₇-C₂₀-Alkylphenyl oder C₁₁-C₂₄-Alkylnaphthyl, sind,
bedeuten, wobei mindestens ein Rest Z ein Rest aus der Gruppe Z², Z⁴ und Z⁶ ist,
stehen.

Von besonderem Interesse sind Verbindungen der allgemeinen Struktur (I), in der
- A: für C₂-C₆-Alkylen, für eine Gruppe der Formel -C₆H₁₀-(C₁-C₄-Alkylen)-C₆H₁₀- oder der Formel -(CₓH₂ₓ-NR′)ₚ-CₓH₂ₓ- ,
in der x 2 bis 4, vorzugsweise 2, und p 1 bis 5, vorzugsweise 1 oder 2, sind und R′ Wasserstoff, C₁-C₄-Alkyl oder eine Gruppe der Formel -(X-O)ₙ- mit einer wie oben definierten freien Valenz bedeutet,
- X: zu 80 bis 100 % für Gruppen der Formeln -CH₂CH₂-,
- B: für C₁-C₆-Alkylen, einen Alkylenrest einer Dicarbonsäure auf Basis dimerisierter ungesättigter C₁₆-C₁₈-Fettsäuren, 1,2-, 1,3- oder 1,4-Phenylen, eine Gruppe der Formel -CH=CH- oder -CH₂CH(SO₃M)-, worin M für ein Kation steht, und
- Z: für gleiche oder verschiedene Reste Z¹ bis Z⁶, worin
- Z¹, Z², Z⁴ und Z⁵: die obengenannte Bedeutung haben und
- Z³: Phenyl, Naphthyl, ein ein- bis dreifach substituierter Phenyl- oder Naphthylrest, wobei die Substituenten aus der Reihe C₁-C₄-Alkyl, C₁-C₄-Alkoxy, Hydroxy, Amino, Acetamido oder Acetoacetamido ausgewählt sind, und
- Z⁶: einen Acylrest der Formel -CO-B-COO-(X-O)ₘ-Y-R³ , in der X, Y und B die oben definierte Bedeutung haben, und m 1 bis 50, vorzugsweise 1 bis 20 ist und -Y-R³ ein Acylrest einer C₁₂-C₁₈-Fettsäure, Phenyl, Naphthyl, C₇-C₁₈-Alkylphenyl, C₁₁-C₂₂-Alkylnaphthyl oder ein Acylrest einer monofunktionellen Harzsäur vom Kolophoniumtyp ist,
bedeuten, wobei mindestens ein Rest Z die Bedeutung von Z², Z⁴ oder Z⁶ hat,
stehen.

Besonders bevorzugte Verbindungen der allgemeinen Struktur (I) sind solche, in denen Z zu mehr als 25 %, insbesondere mindestens 50 % aus Resten der Formeln Z², Z³, Z⁴, Z⁵ und Z⁶ ausgewählt ist.

Die Erfindung betrifft auch ein Verfahren zur Herstellung der erfindungsgemäßen Verbindungen mit der Struktur (I), dadurch gekennzeichnet, daß man Diamin-oxalkylate der Formel (Ia), in der die freien Valenzen durch Z¹ = Wasserstoff abgesättigt sind, teilweise oder ganz mit Verbindungen der Formel H-Z, wobei Z eine oder mehrere der obengenannten Bedeutungen Z² bis Z⁶ und mindestens eine davon die Bedeutung Z², Z⁴ oder Z⁶ hat, in einer oder mehreren Stufen unter Veresterung umsetzt.

Die für das erfindungsgemäße Verfahren verwendeten Aminoxalkylate (Blockpolymerisate) der Formel (Ia) können nach üblichen Methoden aus Diaminen oder Polyaminen durch Umsetzung mit Ethylenoxid und/oder Propylenoxid hergestellt werden oder sind vielfach als solche im Handel erhältlich. Bevorzugte Blockpolymerisate basieren auf aliphatischen oder cycloaliphatischen Diaminen und Polyaminen, wie beispielsweise 1,2-Diaminoethan, 1,3-Diaminopropan, 1,2-Diaminopropan, 1,4-Diaminobutan, 1,5-Diaminopentan, 1,6-Diaminohexan, 1,7-Diaminoheptan, 1,8-Diaminooctan, 1,9-Diaminononan, 1,10-Diaminodecan, 1,12-Diaminododecan, insbesondere die genannten Diamine mit einer geraden Zahl an C-Atomen sowie 1,3-Diaminopropan und 1,5-Diaminopentan, oder deren Mischungen sowie Bis-(4-amino-cyclohexyl)-methan, 1,1-Bis-(4-amino-cyclohexyl)-ethan, 2,2-Bis-(4-amino-cyclohexyl)-propan, Diethylentriamin, Dipropylentriamin, Triethylentetramin, Dipropylentetramin, Tetraethylenpentamin, 2-Methylamino-ethylamin, 2-Ethylamino-ethylamin, 3-Methylamino-propylamin, N-Methyl-dipropylentriamin oder deren Mischungen.

Die Oxalkylierung der Amine zur Herstellung der Basis-Blockpolymerisate (Ia) erfolgt nach üblichen Methoden, vorzugsweise mit Alkalihydroxiden oder -alkoxiden als Katalysator, bei 100 - 200°C, insbesondere bei 140 - 180°C. Die Menge an Ethylenoxid oder Propylenoxid oder einer Mischung beider Epoxide wird so bemessen, daß vorzugsweise eine stabile Emulgierbarkeit oder eine völlige Löslichkeit der gebildeten Basis-Blockpolymerisate in Wasser erreicht wird. Zweckmäßig wird pro reaktionsfähige Stelle im DiaminMolekül, d. h. pro Wasserstoffatom der vorhandenen primären und sekundären Aminogruppen, jeweils 1 bis 150, vorzugsweise 1 bis 50, insbesondere 5 bis 25 Moleküle Epoxid eingesetzt. Die Menge des angelagerten Alkylenoxids bemißt sich auch nach dem beabsichtigten Einsatzzweck und damit nach dem angestrebten Grad der Hydrophilie.

Als Alkalihydroxid eignen sich Kaliumhydroxid oder bevorzugt Natriumhydroxid, als Alkalialkoxid Natriummethylat oder -ethylat. Die Konzentration der alkalischen Katalysatoren soll bevorzugt bei 0,05 - 1,0 Gew.-%, bezogen auf das Amin, bei Beginn der Oxalkylierung sein. Die Oxalkylierung kann drucklos oder in Druckgefäßen mit Propylenoxid oder bevorzugt mit Ethylenoxid oder Mischungen von beiden durchgeführt werden, wobei das Alkylenoxid gasförmig oder flüssig zugeführt werden kann. Der Arbeitsdruck beträgt in der Regel 1 - 10, vorzugsweise 2 - 4 bar.

Die eigentliche Umsetzung zur Erzeugung der erfindungsgemäß modifizierten Blockpolymerisate der allgemeinen Struktur (I) aus den Basis-Blockpolymerisaten (Ia) erfolgt in ein oder mehreren Reaktionsstufen, wobei die Einführung der Reste Z mit der Bedeutung Z², Z³, Z⁴ und Z⁵ in der Regel durch Umsetzung mit entsprechenden Carbonsäuren der Formel R¹-COOH, R²-COOH, Harzsäuren bzw. Dicarbonsäuren der Formel HOOC-B-COOH oder deren Anhydriden vorgenommen wird.

Beispielsweise kann man auf diese Art einen Teil oder alle Hydroxygruppen der oxalkylierten Aminverbindungen (Basis-Blockpolymerisate) mit den vorstehend genannten Carbonsäuren verestern. Ebenso ist es möglich, Gemische solcher Carbonsäuren einzusetzen. In der Regel ist es vorteilhaft, monofunktionelle Carbonsäuren in einer ersten Reaktionsstufe zur Veresterung eines Teils der vorhandenen Hydroxygruppen zu benutzen und danach in einer zweiten Reaktionsstufe mit Dicarbonsäuren zu verestern, wobei in der zweiten Reaktionsstufe je nach angewendeter Menge an Dicarbonsäure anionische Reste vom Typ freier Carboxylatgruppen eingeführt werden, oder, vor allem bei einem Unterschuß an Dicarbonsäure, eine Vernetzung der Basismoleküle über die Dicarbonsäuren stattfindet.

Als Carbonsäuren der Formel R¹-COOH eignen sich beispielsweise gesättigte oder ungesättigte Carbonsäuren oder Hydroxycarbonsäuren mit 8 bis 22 C-Atomen, wie beispielsweise Octansäure, Pelargonsäure, Caprinsäure, Undecansäure, Laurinsäure, Tridecansäure, Myristinsäure, Pentadecansäure, Palmitinsäure, Heptadecansäure, Stearinsäure, Nonadecansäure, Arachinsäure, Behensäure, 10-Undecensäure, Lauroleinsäure, Myristoleinsäure, Palmitoleinsäure, 6c- und 6t-Octadecensäure, Elaidinsäure, Ölsäure, Linolsäure, Linolensäure, Rizinolsäure oder Rizinensäure, insbesondere geradzahlige Fettsäuren oder Hydroxyfettsäuren mit jeweils 8 bis 20 C-Atomen, beispielsweise die entsprechend vorstehend genannten geradzahligen Fettsäuren sowie insbesondere ihre aus Naturprodukten gewonnenen Gemische, wie Tallölfettsäure, Talgfettsäure, Kokosölfettsäure, Palmölfettsäure, Leinölfettsäure, Rizinusölfettsäure und Rizinensäuren, vorzugsweise die genannten Fettsäuren mit 12 bis 18 C-Atomen; weiterhin geeignet sind modifizierte Fettsäuren und deren Gemische, wie sie durch Friedel-Crafts-Reaktion von aromatischen Hydroxyverbindungen, beispielsweise Phenol, o-, m- und p-Kresol, Guayacol, Salicylsäure, α-Naphthol oder β-Naphthol, mit ungesättigten Fettsäuren, wie Palmitoleinsäure, Ölsäure, Undecylsäure und Rizinolsäure, in Gegenwart von stark sauren oder säureabspaltenden Katalysatoren, wie z. B. Bortrifluorid, Aluminiumchlorid, p-Toluolsulfonsäure, Methansulfonsäure, Mineralsäuren oder Ionenaustauschern bei Temperaturen zwischen 50 - 200°C, vorzugsweise 120 - 160°C, ggfs. im organischen Medium, wobei auf 1 Mol der genannten Phenole oder Naphthole 0,5 - 1,1 Mol, vorzugsweise 0.9 - 1 Mol der ungesättigten Fettsäuren verwendet werden, erhältlich sind.

Als Carbonsäuren R²-COOH eignen sich beispielsweise Monocarbonsäuren mit 2 bis 6 C-Atomen, wie Essigsäure, Propionsäure, Buttersäure, Valeriansäure, Capronsäure und Heptansäure, weiterhin aromatische Carbonsäuren, wie Benzoesäure, Anthranilsäure, p-Aminobenzoesäure, Salicylsäure, o-, m- und p-Tolylsäuren, -Methoxy- und -Ethoxybenzoesäuren, -Acetoacetamidobenzoesäuren und -Acetamidobenzoesäuren, Phenylessigsäure oder Naphthoesäuren, insbesondere Oxynaphthoesäuren, beispielsweise 3-Hydroxy-1-naphthoesäure, 3-Hydroxy-2-naphthoesäure, 4-Hydroxy-2-naphthoesäure, 5-Hydroxy-1-naphthoesäure, 5-Hydroxy-2-naphthoesäure, 6-Hydroxy-2-naphthoesäure und 7-Hydroxy-2-naphthoesäure.

Als Carbonsäuren Z⁴-H eignen sich unmodifiziert oder modifizierte Naturharzsäuren vom Kolophoniumtyp oder reaktionsfähige Derivate davon, vorzugsweise Harzsäuren wie Abietinsäure, Dehydroabietinsäure, Tetrahydroabietinsäure, Lävopimarsäure, Dextropimarsäure oder Isodextropimarsäure, wie sie in handelsüblichen Kolophoniumarten vorliegen, sowie modifizierte Harzsäuren, wie disproportionierte, hydrierte und dimerisierte Naturharzsäuren.

Zur Einführung der Gruppen Z⁶ kann man das Basis-Diaminoxalkylat (Ia) oder teilveresterte Diaminoxalkylate mit Carbonsäuren der definierten Formel HOOC-B-COO-(X-O)ₘ-Y-R³ verestern, wobei solche Carbonsäuren insbesondere aus Fettalkoholen oder Addukten von Ethylenoxid und/oder Propylenoxid an Fettsäuren, Fettalkoholen, Phenolen, Naphtholen, Alkylphenolen oder Alkylnaphtholen durch Veresterung mit Dicarbonsäuren zugänglich sind. Es ist auch möglich, wie es weiter unten erwähnt wird, zunächst mit den Dicarbonsäuren durch Halbveresterung anionische Gruppen in das Basis-Oxalkylat einzuführen und die freien Carboxygruppen dann mit Fettalkoholen, oxalkylierten Fettsäuren oder oxalkylierten Fettalkoholen umzusetzen.

Die Verknüpfung mehrerer Blockpolymerisateinheiten der Formel (Ia) kann durch Umsetzung der Basisblockpolymerisate in einer ersten Reaktionsstufe durch Veresterung mit einer oder mehreren Dicarbonsäuren der Formel HOOC-B-COOH im Molverhältnis von 2 : 1 bis 10 : 9, vorzugsweise 2 : 1 bis 5 : 4, erfolgen. Als Dicarbonsäuren eignen sich zur Verknüpfung vorzugsweise aliphatische Dicarbonsäuren mit 3 - 12 Kohlenstoffatomen, insbesondere Malonsäure, Bernsteinsäure, Glutarsäure, Adipinsäure, 1,5-Pentandicarbonsäure, 1,6-Hexandicarbonsäure oder 1,10 Decandicarbonsäure, aber auch beispielsweise Cyclohexan-1,4-dicarbonsäure und aromatische Dicarbonsäuren wie Phthalsäure oder Terephthalsäure.

Anstelle der Carbonsäuren können zur Veresterung auch, soweit vorhanden, die Anhydride oder reaktive Carbonsäurederivate eingesetzt werden, beispielsweise im Falle von Umesterungsreaktionen andere Ester der Carbonsäuren.

Die erfindungsgemäße Verknüpfung zweier oder mehrerer Blockpolymerisateinheiten, die schon an einem Teil der Polyglykoletherketten endständig mit Monocarbonsäuren verestert sind, kann vorzugsweise in einer zweiten Reaktionsstufe durch Veresterung mit den obengenannten Dicarbonsäuren durchgeführt werden, wobei vorzugsweise wiederum ein Verhältnis von Blockpolymerisat zu Dicarbonsäure von 2 : 1 bis 10 : 9 gewählt wird, ausgenommen von dem Fall, in dem das teilveresterte Basis-Blockpolymerisat nur noch eine freie Hydroxygruppe besitzt. In dem letztgenannten Fall ist nur ein Molverhältnis von etwa 2 : 1 sinnvoll.

Es ist allerdings auch möglich, die Umsetzung der Basis-Blockpolymerisate mit Monocarbonsäuren und Dicarbonsäuren in einer Reaktionsstufe durchzuführen, wobei dann in der Regel Gemische von mehrkernigen und einkernigen veresterten Blockpolymerisaten entstehen.

Die Veresterung der Basis-Blockpolymerisate (Ia) mit den Monocarbonsäuren oder Dicarbonsäuren kann nach an sich üblichen Veresterungsmethoden erfolgen. Die einzuhaltende Reaktionstemperatur liegt dabei in der Regel zwischen Raumtemperatur und 240°C, je nach Veresterungsmethode. Bevorzugt wird zur Erhöhung der Ausbeute die Veresterung in einem inerten organischen Lösungsmittel durchgeführt, das als Schleppmittel zum Entfernen des Reaktionswassers geeignet ist. Beispielsweise kann die Veresterung in Xylol als organischem Lösungsmittel und in Gegenwart von sauren Katalysatoren bei einer Temperatur von 130 - 220°C durchgeführt werden. Als saure Katalysatoren sind beispielsweise Säuren und Lewissäuren wie Benzolsulfonsäure, p-Toluolsulfonsäure, Borsäure, Zinnpulver, Zinkchlorid oder Schwefelsäure möglich.

Die Veresterung der Basis-Blockpolymerisate kann alternativ auch durch Umesterung unter Einsatz der entsprechenden Alkylester, vorzugsweise Methylester der genannten Monocarbonsäuren oder Dicarbonsäuren meist in Gegenwart von Katalysatoren, vorzugsweise in Gegenwart von 0,1 - 1,0-molaren Menge eines Alkoxylats pro Mol Ester, vorzugsweise Natriummethylat bei 150 - 200°C, insbesondere 160 - 190°C, unter Abdestillieren des freiwerdenden Alkanols bzw. Methanols, erfolgen. Die in der ersten oder zweiten Reaktionsstufe erhaltenen veresterten Blockpolymerisate sind als solche wertvolle oberflächenaktive Mittel und können im Sinne der Erfindung eingesetzt werden.

Zur Einführung von anionischen Gruppen können die Basis-Blockpolymerisate oder die teilweise mit Monocarbonsäuren veresterten Blockpolymerisate, sofern letztere noch freie Hydroxygruppen aufweisen, beispielsweise mit Schwefelsäure, Amidosulfonsäure oder Chlorsulfonsäure oder entsprechenden Anhydriden umgesetzt werden, wobei Sulfatogruppen eingeführt werden. Nach einer anderen Variante werden die Basis-Blockpolymerisate oder die teilveresterten Blockpolymerisate mit einem Überschuß an Dicarbonsäuren oder deren Anhydriden zu den entsprechenden Halbestern umgesetzt. Als Dicarbonsäuren eignen sich die bereits obengenannten zur Vernetzung der Blockpolymerisateinheiten der Formel (Ia) geeigneten Dicarbonsäuren der Formel HOOC-B-COOH und deren Anhydride.

Vorzugsweise werden die anionischen Gruppen durch Reaktion mit Maleinsäureanhydrid oder Phthalsäureanhydrid durch Mischen und Verrühren bei 20 - 100°C, bevorzugt bei 40 - 80°C, in Anwesenheit von Alkalihydroxiden umgesetzt. Die Konzentration der Alkalihydroxide soll vorzugsweise 0,1 - 1,0 Gew.-%, bezogen auf die Gesamtmischung, betragen. Im Fall von Maleinsäureanhydrid ist es wegen der Sublimationsneigung vorteilhaft, in Druckgefäßen unter einem Überdruck von 0,2 - 1 bar Stickstoff oder Luft zu arbeiten und für kräftiges Durchmischen zu sorgen, da zu Beginn der Reaktion das geschmolzene Maleinsäureanhydrid mit den teilveresterten Oxalkylaten schlecht mischbar ist.

Im Fall von eingeführten Maleinsäurehalbestergruppen ist es außerdem vorteilhaft, diese Halbestergruppen in die entsprechenden Sulfobernsteinsäurehalbestergruppen zu überführen. Dies gelingt beispielsweise durch Zugabe wäßriger Lösungen von Sulfiten oder Hydrogensulfiten zu den Verbindungen, die Maleinsäurehalbestergruppen aufweisen. Pro Mol Maleinsäurehalbestergruppe werden 1,0 - 1,5, vorzugsweise 1,0 - 1,1 Mol schweflige Säure in Form von Alkali- oder Erdalkalisulfiten oder -hydrogensulfiten oder -pyrosulfiten eingesetzt. Die Umsetzung wird in der Regel in Gegenwart von etwa 50 - 85 % Wasser, bezogen auf die gesamte Lösung bzw. Mischung, durchgeführt. Die Wassermenge ist abhängig von der Löslichkeit der zugrundeliegenden Sulfobernsteinsäurehalbestersalze und der Viskosität der Lösungen. Die Reaktionstemperatur bei der Umsetzung von Sulfiten mit den Maleinsäurehalbesterverbindungen beträgt in der Regel 20 - 100°C, insbesondere 40 - 80°C.

Während sich die Sulfite besonders zur Bildung der Dialkalisalze der Sulfobernsteinsäurehalbester eignen, ist es bei der Anlagerung von Hydrogensulfiten möglich, durch Neutralisation mit Basen wie Ammoniak, niedermolekularen Alkylaminen oder Alkylolaminen oder von entsprechenden Alkylenoxidaddukten, wobei pro Mol Amin oder Alkylolamin bis zu etwa 150 Mol Ethylenoxid oder Propylenoxid oder beide Alkylenoxide angelagert und pro reaktionsfähiges Wasserstoffatom der genannten Verbindungen bis zu 150, vorzugsweise 5 - 30 Moleküle Ethylenoxid oder Propylenoxid oder beide angelagert sind, den Grad den Hydrophilie zusätzlich zu beeinflussen. Als Vertreter der Alkylamine oder Alkylolamine seien genannt Ethylamin, Propylamin, Isopropylamin, Butylamin, Isobutylamin, Monoethanolamin, Monopropanolamin, Monoisopropanolamin, Monobutanolamin, Monoisobutanolamin, Diethanolamin, Dipropanolamin, Dibutanolamin, Triethanolamin, Tripropanolamin oder Tributanolamin sowie Di- und Polyamine wie Ethylendiamin, Ethylentriamin, Triethylentetramin, Propylendiamin, Dipropylendiamin, Dipropylentriamin oder Tripropylentetramin.

Der am Beispiel der Sulfobernsteinsäurehalbestergruppen beschriebene Kationenaustausch kann auch bei den Verbindungen der Struktur (I) erfolgen, die andere anionische Gruppen aufweisen. Dabei werden die Verbindungen in ihrer Säureform eingesetzt und in analoger Weise durch Neutralisation mit den obengenannten Aminen oder anorganischen Basen in die entsprechenden Salze überführt. Auch andere übliche Kationenaustauschverfahren können angewendet werden.

Während bei der Sulfatierung mit Amidosulfonsäure die Ammoniumsalze der Schwefelsäurehalbester anfallen, entstehen bei der technisch interessantesten Ausführungsform mit gasförmigen Schwefeltrioxid in Mischungen mit Inertgas sowie auch bei der Sulfatierung mit Chlorsulfonsäure Schwefelsäurehalbester in der Säureform, aus denen leicht die gewünschten Salze durch Neutralisation mit entsprechenden anorganischen oder organischen Basen hergestellt werden können. Zu dieser Neutralisation werden bevorzugt die Alkalihydroxyide eingesetzt, die zu den sehr gut wasserlöslichen Alkalisalzen der erfindungsgemäßen Schwefelsäurehalbester führen.

In einer weiteren Reaktionsstufe ist es möglich, modifizierte Blockpolymerisate, die noch anionische Reste in Form von Carboxylatresten aufweisen, durch Umsetzung mit Alkoholen der Formel HO-(X-O)ₘ-Y-R² zu modifizierten Blockpolymerisaten umzusetzen, in denen Z die obengenannte Bedeutung von Z⁶ hat. Diese Veresterungsreaktion kann analog den bereits obengenannten Veresterungen durchgeführt werden.

Die erfindungsgemäßen Verbindungen weisen vielseitige vorteilhafte Eigenschaften auf. Sie gehören zu der Klasse der oberflächenaktiven Verbindungen nach DIN 53900, senken die Oberflächenspannung nach der Ringabreißmethode (DIN 53914) und sind nach den Resultaten im modifizierten Ross-Miles-Test (DIN 53902) als nichtschäumende bzw. schwachschäumende oberflächenaktive Stoffe zu bezeichnen. Bei geeignetem Hydrophilierungsgrad zeigen sie ausgezeichnetes Netzvermögen für Baumwolle nach der Tauchnetzmethode (DIN 53901) bei gleichzeitig gutem Egalisierverhalten nach DIN 53504. Sie besitzen ein sehr gutes Flockungsschutzvermögen gegenüber Pigmenten und Farbstoffen (DIN 53908) und eine sehr gute Wasserverteilungswirkung als Reinigungsverstärker (DIN 53980) sowie eine gute Auswaschbarkeit als Schmelzmittel (DIN 53504). Die Produkte sind biologisch abbaubar.

Die erfindungsgemäßen Substanzen können aufgrund ihrer vielseitigen oberflächenaktiven Eigenschaften für ein breites Spektrum von Anwendungen eingesetzt werden.

Gegenstand der Erfindung ist deshalb auch die Verwendung der Verbindungen der obengenannten Struktur (I) als oberflächenaktive Mittel. Von besonderem Interesse ist die Verwendung für den Einsatz als Kupplungshilfsmittel und Emulgator bei der Herstellung von Azofarbmitteln, insbesondere von Azopigmenten, als Dispergiermittel und Verteilungsmittel für die Feinverteilung und Stabilisierung von schwerlöslichen oder unlöslichen Farbmitteln, vorzugsweise für die Herstellung von gut fließbaren Pigmentdispersionen für den wäßrigen Druckfarbensektor oder zur Herstellung von Präparationen von Dispersionsfarbstoffen, wie sie bevorzugt zum Färben von natürlichen und synthetischen Fasermaterialien, wie Baumwolle, Wolle, Zellulose, Zellwolle, Zelluloseacetat, Zellulosetriacetat, Polyester, Polyamid und Polyacrylnitril, oder von Fasermaterialien, die diese Stoffe enthalten, verwendet werden. Die erfindungsgemäßen Substanzen eignen sich als Additive und Emulgatoren, wie z. B. als Korrosionsschutzzusätze, als Zusätze für die Herstellung von Kühlschmiermitteln und Kaltwalzölen in der Metallverarbeitungsindustrie. Weiterhin können die Substanzen als Dispergier- und Emulgiermittel für die Herstellung von Reinigungsverstärkern, Carrier-Emulsionen und Formulierungen für Pflanzenschutz- und Schädlingsbekämpfungsmitteln verwendet werden. Sie eignen sich ebenfalls als Netz- und Egalisiermittel in der Färberei.

Die Verbindungen können einzeln, als Gemische oder in Kombination mit anderen nichtionogenen, anionaktiven und/oder kationaktiven Tensiden, Gerüstsubstanzen und anderen üblichen Zusätzen oder Hilfsstoffen zur Anwendung kommen.

Die nachfolgenden Beispiele dienen zur näheren Erläuterung der Erfindung. Teile und Prozentangaben beziehen sich auf das Gewicht, Druckangaben sind Überdruck, bezogen auf Atmosphärendruck, sofern nichts anderes angegeben ist. Volumenteile verhalten sich zu Gewichtsteilen wie Liter zu Kilogramm.

Zur Charakterisierung der hergestellten Verbindungen ist in mehreren Beispielen die Hydroxylzahl oder die Säurezahl angegeben. Die Hydroxylzahl ist die Menge KOH in mg, welche notwendig ist, um die Menge an Essigsäure zu neutralisieren, die bei der Acetylierung von 1 g der Verbindung verbraucht wird. Die Säurezahl ist die Menge an KOH in mg, die zur Neutralisation von 1 g der Verbindung benötigt wird.

### Herstellungsbeispiele

### 1a) Diaminoethan + 32 EO

50 Teile 1,2-Diaminoethan werden unter Rühren und Zuführen von 1 173 Teilen Ethylenoxid (EO) ohne Zugabe eines Katalysators unter Aufrechterhalten eines Drucks von 2 - 4 bar bei einer Anfangstemperatur von 80°C und einer Endtemperatur von 140°C oxethyliert. Nachdem alles Ethylenoxid in das Reaktionsgefäß gedrückt worden ist, läßt man noch eine Stunde bei 130 - 140°C rühren. Das erhaltene Produkt hat eine Hydroxylzahl von 155.

### 1b) Ester von 1a) mit 3 Moläquivalenten Talgfettsäure

146,8 Teile Oxethylat 1a) werden mit 83,7 Teilen handelsüblicher Talgfettsäure auf 70 - 80°C erwärmt und unter Stickstoffgas eine Stunde lang gerührt. Nach Zugabe von 1,5 Teilen p-Toluolsulfonsäure und 150 Teilen Xylol wird 16 Stunden lang auf 150 - 160°C erhitzt, wobei das Reaktionswasser durch Auskreisen am Wasserabscheider entfernt wird. Danach wird das Xylol abdestilliert und die Säurezahl nach DIN 53185 bestimmt. Das Produkt hat eine Säurezahl von weniger als 20.

### 2) Ester von 1a) mit 2 Moläq. Kolophonium

440,4 Teile Oxethylat 1a) werden mit 181,2 Teilen disproportioniertem Kolophonium auf 70 - 80°C erwärmt und unter Stickstoffgas eine Stunde lang gerührt. Nach Zugabe von 12 Teilen Zinnpulver, 4,0 Teilen p-Toluolsulfonsäure und 150 Vol.-Teilen Xylol wird 16 Stunden lang auf 150 - 160°C erhitzt und das Reaktionswasser durch Auskreisen entfernt. Nach Abdestillieren des Xylols erhält man ein Produkt mit einer Säurezahl von weniger als 25.

### 3) Mischester von 1a) mit 2 Moläq. Talgfettsäure und 2 Moläq. Phthalsäure (Halbester)

146,8 Teile Oxethylat 1a) werden analog 1b) mit 55,8 Teilen handelsüblicher Talgfettsäure bis zu einer Säurezahl von 16 verestert und anschließend in Gegenwart von 0,2 Teilen pulverisiertem Ätznatron mit 29,6 Teilen Phthalsäureanhydrid innerhalb von 5 Stunden bei 80 - 90°C umgesetzt. Durch Zugabe einer Lösung von 440 Teilen Wasser und 16 Teilen Ätznatron wird anschließend auf pH 6,8 - 7,0 eingestellt. Die Menge des zugesetzen Wassers liegt vorzugsweise zwischen 50 und 85 % der fertigen Produktlösung.

### 4) Mischester auf Basis von 1a), 2 Moläq. Talgfettsäure und 2 Moläq. Sulfobernsteinsäurehalbestergruppen

146,8 Teile Oxethylat 1a) werden analog Verbindung 1b) mit 83,7 Teilen handelsüblicher Talgfettsäure bis zu einer Säurezahl von kleiner als 15 verestert und anschließend in Gegenwart von 0,1 Teilen pulverisiertem Ätznatron mit 19,2 Teilen Maleinsäureanhydrid innerhalb von 4 Stunden bei 70 - 80°C verestert und anschließend durch Zugabe einer Lösung von 24,7 Teilen Natriumsulfit in 508 Teilen Wasser zum Sulfosuccinat umgesetzt.

### 5a) Diaminoethan + 4 PO + 24 EO

50 Teile 1,2-Diaminoethan werden unter Rühren und Zuführen von 183,3 Teilen Propylenoxid (PO) bei 90 - 110°C oxalkyliert und nach Zugabe von 3 Teilen Natriumethylat und Entfernen des Methanols unter reduziertem Druck mit 879,6 Teilen Ethylenoxid bei 120 - 140°C bei 3 - 5 bar umgesetzt. Das erhaltene Produkt hat eine Hydroxylzahl von etwa 163.

### 5b) Ester aus 5a) und 2 Moläq. Rizinenfettsäure und 2 Moläq. Phthalsäure

137,8 Teile Oxalkylat 5a) wurden mit 56 Teilen handelsüblicher Rizinenfettsäure analog Verbindung 1b) bis zu einer Säurezahl von kleiner als 18 verestert und anschließend in Gegenwart von 0,2 Teilen pulverisiertem Ätznatron mit 29,6 Teilen Phthalsäureanhydrid innerhalb von 5 Stunden bei 80 - 90°C umgesetzt. Durch Zugabe einer Lösung von 764 Teilen Wasser und 188,2 Teilen eines Addukts aus Triethanolamin und 18 Moläq. Ethylenoxid wird ein pH-Wert von 6,8 - 7,0 eingestellt.

### 6) Ester aus 5a) und 1 Moläq. Tallölfettsäure und 1 Moläq. 2-Acetoacetamido-benzoesäure

137,8 Teile Oxethylat 5a) werden mit 28,2 Teilen handelsüblicher Tallölfettsäure entsprechend 1b) bis zu einer Säurezahl von weniger als 20 verestert, ohne das Xylol am Ende abzudestillieren. Nach Zugabe von weiteren 0,5 Teilen p-Toluolsulfonsäure und 22,1 Teilen 2-(Acetoacetamido)-benzoesäure wird analog der vorigen Veresterung bis zu einer Säurezahl von kleiner als 25 verestert und das Xylol am Ende der Veresterung abdestilliert.

### 7a) Oxethylat aus Diaminoethan + 8 PO + 60 EO

50 Teile 1,2-Diaminoethan werden analog 5a) mit 387 Teilen Propylenoxid und 2 200 Teilen Ethylenoxid im Temperaturbereich von 80 - 140°C bei 2 - 4 bar oxalkyliert. Gegen Ende der Reaktion läßt man noch eine Stunde bei 135 - 140°C rühren. Das Produkt hat eine Hydroxylzahl von etwa 71.

### 7b) Ester aus 7a) und 1 Moläq. Talgfettsäure und 1 Moläq. 2-Hydroxy-3-naphthoesäure

300 Teile Oxalkylat 7a) werden mit 25,5 Teilen einer handelsüblichen Talgfettsäure entsprechend Verbindung 1b) in Xylol bis zu einer Säurezahl von kleiner als 10 verestert. Nach Zugabe von weiteren 0,7 Teilen p-Toluolsulfonsäure und 17,8 Teilen 2-Hydroxy-3-naphthoesäure wird unter Auskreisen des Reaktionswassers bis zu einer Säurezahl von weniger als 20 verestert. Anschließend wird das Xylol abdestilliert.

### 8) Ester aus 7a) und 1 Moläq. Kolophonium und 1 Moläq. 2-Hydroxy-6-naphthoesäure

300 Teile Oxalkylat 7a) werden mit 28,7 Teilen handelsüblichem disproportionierten Kolophonium entsprechend Verbindung 2) in Xylol bis zu einer Säurezahl von kleiner als 20 verestert. Nach Zugabe von 0,5 Teilen p-Toluolsulfonsäure und 17,8 Teilen 2-Hydroxy-6-naphtoesäure wird analog Verbindung 7b) bis zu einer Säurezahl von weniger als 18 weiterverestert und anschließend das Xylol abdestilliert.

### 9a) Diaminopropan + 4 PO + 32 EO

50 Teile 1,3-Diaminopropan werden analog Verbindung 5a) mit 156,8 Teilen Propylenoxid bei 90 - 110°C oxpropyliert und nach Zugabe von 2 Teilen Natriummethylat das entstehende Methanol unter reduziertem Druck entfernt. Anschließend setzt man mit 951,3 Teilen Ethylenoxid bei 120 - 140°C und 2 - 4 bar um. Das erhaltene Oxalkylat besitzt eine Hydroxylzahl von etwa 131.

### 9b) Ester aus 9a) und 1 Moläq. Talgfettsäure und 1 Moläq. Kolophonium

300 Teile 9a) werden mit 47,5 Teilen handelsüblicher Talgfettsäure und 59,8 Teilen disproportioniertem Kolophonium nach Zugabe von 6 Teilen Zinnpulver, 1,5 Teilen p-Toluolsulfonsäure und 150 Vol.-Teilen Xylol bei 155 - 165°C innerhalb von 16 Stunden unter Auskreisen des Reaktionswassers verestert. Nach Abdestillieren des Xylols erhält man ein Produkt mit einer Säurezahl von weniger als 20.

### 10a Oxalkylat aus Diethylentriamin + 10 PO + 40 EO

50 Teile Diethylentriamin werden analog Verbindung 6a) mit 281,3 Teilen Propylenoxid und nach Zugabe von 2,5 Teilen Natriummethylat und Entfernen des Methanols unter reduziertem Druck mit 853,3 Teilen Ethylenoxid umgesetzt. Das Produkt hat eine Hydroxylzahl von etwa 115.

### 10b) Ester aus 10a) und 3 Moläq. Tallölfettsäure

500 Teile Oxalkylat 10a) werden mit 173 Teilen handelsüblicher Tallölfettsäure gemäß Verbindung 1b) nach Zugabe von 3 Teilen Borsäure und 200 Teilen Xylol bei 155 - 160°C innerhalb von 12 Stunden verestert. Nach Abdestillieren des Xylols erhält man ein Produkt mit einer Säurezahl von weniger als 18.

### 11) Ester auf Basis von 10a) und 2 Moläq. Tallölfettsäure und 2 Moläq. Sulfobernsteinsäurehalbestergruppen

300 Teile Ester 10b) werden in Gegenwart von 0,1 Teilen pulverisiertem Ätznatron mit 24 Teilen Maleinsäureanhydrid innerhalb von 4 Stunden bei 70 - 80°C verestert und durch Zugabe einer Lösung aus 30,9 Teilen Natriumsulfit in 657 Teilen Wasser innerhalb von 3 Stunden bei 75 - 85°C umgesetzt.

### 12a) Bis-(4-aminocyclohexyl)-methan + 40 EO

103 Teile Bis-(4-aminocyclohexyl)-methan werden gemäß Beispiel 5a) mit 116 Teilen Propylenoxid und nach Zugabe von 2 Teilen Natriummethylat mit 870 Teilen Ethylenoxid umgesetzt. Das erhaltene Produkt hat eine Hydroxylzahl von 105.

### 12b) Ester aus 12a) und 2 Moläq. Talgfettsäure

500 Teile Oxalkylat 12a) werden mit 123,5 Teilen handelsüblicher Talgfettsäure analog Beispiel 1b) nach Zugabe von 3 Teilen p-Toluolsulfonsäure und 200 Vol.-Teilen Xylol bei 155 - 165°C innerhalb von 15 Stunden verestert.

Nach Abdestillieren des Xylols erhält man ein Produkt mit einer Säurezahl von weniger als 15.

### 13) Ester auf Basis 12a) und 2 Moläq. Talgfettsäure und 2 Moläq. Sulfobernsteinsäurehalbestergruppen

300 Teile 12b) werden in Gegenwart von 0,1 pulverisiertem Ätznatron mit 28 Teilen Maleinsäureanhydrid innerhalb von 3 Stunden bei 70 - 80°C verestert und durch Zugabe einer Lösung aus 34,7 Teilen Natriumsulfit in 674 Teilen Wasser innerhalb von 3 Stunden bei 75 - 85°C umgesetzt.

### 14) Ester auf Basis von Malonsäure und 3 Moläq. 5a)

600 Teile Oxalkylat 5a) werden mit 30,9 Teilen Malonsäure bei 70 - 80°C unter Stickstoffgas erwärmt. Nach Zugabe von 3 Teilen p-Toluolsulfonsäure und 200 Vol.-Teilen Xylol 10 Stunden lang auf 155 - 165°C erhitzt und das Reaktionswasser durch Auskreisen entfernt. Nach Abdestillieren des Xylols hat das Produkt eine Säurezahl von weniger als 10, eine Hydroxylzahl von etwa 106 und enthält pro Molekül 12 Propylenoxid-Einheiten und 72 Ethylenoxid-Einheiten.

### 15) Ester aus 14) und 8 Moläq. Ölsäure

300 Teile Oxalkylat 14) werden mit 159 Teilen handelsüblicher Ölsäure analog 1b) nach Zugabe von 2,1 Teilen p-Toluolsulfonsäure und 150 Vol.-Teilen Xylol bei 155 - 165°C innerhalb von 18 Stunden verestert. Nach Abdestillieren erhält man ein Produkt mit einer Säurezahl von weniger als 25.

### 16) Ester auf Basis 14) und 4 Moläq. Ölsäure und 4 Moläq. Sulfobernsteinsäurehalbestergruppen

300 Teile Verbindung 14) werden mit 80 Teilen handelsüblicher Ölsäure analog 1b) bis zu einer Säurezahl von weniger als 10 verestert. Anschließend wird in Gegenwart von 0,1 Teilen pulverisiertem Ätznatron mit 27,7 Teilen Maleinsäureanhydrid innerhalb von 3 Stunden bei 70 - 80°C weiter verestert und anschließend durch Zugabe einer Lösung von 35,6 Teilen Natriumsulfit in 824 Teilen Wasser zum Sulfobernsteinsäurehalbester umgesetzt.

### 17) Polyblockpolymerisat auf Basis von Malonsäure und 5 Moläq. 5a)

600 Teile Oxalkylat 5a) werden analog Beispiel 14) mit 37 Teilen Malonsäure bis zu einer Säurezahl von weniger als 8 verestert. Das Produkt hat eine Hydroxylzahl von etwa 100.

### 18) Ester von 17) mit 4 Moläq. Talgfettsäure und 4 Moläq. Kolophonium

300 Teile Verbindung 17) werden mit 45 Teilen handelsüblicher Talgfettsäure und 25,3 Teilen disproportioniertem Kolophonium analog Verbindung 1b) nach Zugabe von 6 Teilen Zinnpulver und 2 Teilen p-Toluolsulfonsäure und 150 Vol.-Teilen Xylol bei 155 - 165°C innerhalb von 16 Stunden verestert. Nach Abdestillieren des Xylols hat das Produkt eine Säurezahl von weniger als 20.

### 19) Ester auf Basis von 17) und 4 Moläq. Talgfettsäure und 3 Moläq. Benzoesäure und 5 Moläq. Sulfobernsteinsäurehalbestergruppen

300 Teile Verbindung 17) werden mit 45,5 Teilen handelsüblicher Talgfettsäure und 21,0 Teilen Benzoesäure analog Verbindung 1b) nach Zugabe von 2 Teilen Zinnpulver und 150 Vol.-Teilen Xylol bei 155 - 165°C in 12 Stunden verestert. Nach Abdestillieren des Xylols hat das Produkt eine Säurezahl von weniger als 25. Anschließend wird in Gegenwart von 0,1 Teilen pulverisiertem Ätznatron und 16,4 Teilen Maleinsäureanhydrid innerhalb von 3 - 4 Stunden bei 70 - 80°C weiterverestert und anschließend durch Zugabe einer Lösung von 21,1 Teilen Natriumsulfit zum Sulfobernsteinsäurehalbester umgesetzt.

### 20a) Ester auf Basis von Adipinsäure und 2 Moläq. Verbindung 7a)

600 Teile Oxalkylat 7a) werden entsprechend Beispiel 14) mit 13,8 Teilen Adipinsäure bis zu einer Säurezahl von weniger als 14 verestert. Das erhaltene Polyblockpolymerisat hat eine Hydroxylzahl von etwa 53 und enthält 16 Propylenoxid-Einheiten und 120 Ethylenoxid-Einheiten pro Molekül.

### 20b) Ester aus 20a) und 4 Moläq. Kolophonium

300 Teile Verbindung 20a) werden mit 55,8 Teilen handelsüblichem Kolophonium nach Zugabe von 8 Teilen Zinnpulver und 1,5 Teilen p-Toluolsulfonsäure und 150 Vol.-Teilen Xylol bei 155 - 165°C innerhalb von 18 Stunden unter Auskreisen des Reaktionswassers verestert. Nach Abdestillieren des Xylols erhält man ein Produkt mit einer Säurezahl von weniger als 30.

### 21a) 1,2-Diaminoethan + 8 PO + 48 EO

1,2-Diaminoethan werden unter Rühren analog Beispiel 5a) unter Zuführen von 386,6 Teilen Propylenoxid und 1760 Teilen Ethylenoxid bei 90 bis 140°C bei 3 bis 5 bar oxalkyliert. Das erhaltene Produkt hat eine Hydroxylzahl von etwa 85.

### 21b) Ester von 21a) mit 1 Moläq. Dimer-Fettsäure

500 Teile Oxalkylat 21a) werden mit 145 Teilen einer handelsüblichen dimerisierten Fettsäure, die etwa 22 Gew.-% trimerisierte Fettsäure enthält, nach Zugabe von 200 ml Xylol und 5 Teilen p-Toluolsulfonsäure bei 150 bis 160°C für 16 Stunden im Rückfluß unter Auskreisen des Reaktionswassers erhitzt. Nach Abdestillieren des Xylols erhält man eine Säurezahl von weniger als 30.

## Patentansprüche

1. Verbindung mit der Struktur eines modifizierten Blockpolymerisates (I), formelmäßig aufgebaut aus
a) 1 bis 10 trivalenten oder tetravalenten BlockoxalkylatEinheiten auf Basis von Diaminen oder Polyaminen (Aminoxalkylate) mit der allgemeinen Formel (Ia) 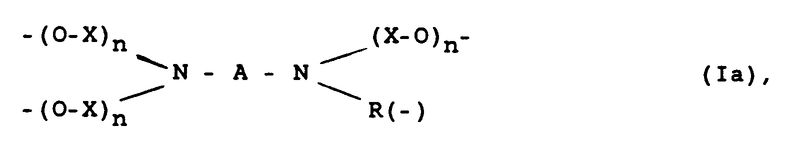
b) monovalenten, durch die allgemeine Formel (Ib)
-Z (Ib)
symbolisierten Säureresten,
und im Falle des Vorhandenseins von zwei oder mehreren Formeleinheiten (Ia) zusätzlich aus
c) divalenten Gruppen der allgemeinen Formel (Ic)
- CO - B - CO - (Ic),
wobei jede der gezeigten freien Valenzen in den Formeleinheiten (Ia) so definiert ist, daß sie unabhängig voneinander mit jeweils einer Formeleinheit (Ib) oder einer Valenz der Formeleinheit (Ic) direkt verbunden ist, und wobei in den Formeleinheiten (Ia) bis (Ic)
A für einen geradkettigen, verzweigten oder cyclischen aliphatischen Rest, einen aromatischen oder araliphatischen Rest mit jeweils 1 bis 300 C-Atomen,
X für gleiche oder verschiedene Gruppen der Formeln -CH₂CH₂- und -CH₂CH(CH₃)- ,
n für gleiche oder verschiedene Zahlen von 1 bis 100,
R für eine C₁-C₃-Alkylgruppe oder eine divalente Gruppe der Formel -(X-O)ₙ- mit einer wie oben definierten, durch (-) angedeuteten freien Valenz,
B für einen geradkettigen, verzweigten oder cyclischen aliphatischen Rest, einen aromatischen oder araliphatischen Rest mit jeweils 1 bis 60 C-Atomen,
Z für gleiche oder verschiedene Reste Z¹ bis Z⁶, worin
Z¹ Wasserstoff,
Z² einen Acylrest der Formel R¹-CO-, in der R¹ ein geradkettiger, gesättigter oder ungesättigter C₇-C₂₁-aliphatischer KW-Rest, der noch durch ein oder zwei Hydroxygruppen oder durch einen C₆-C₁₂-Aryl- oder C₆-C₁₂-Hydroxyarylrest substituiert sein kann,
Z³ einen Acylrest der Formel R²-CO-, in der R² einen geradkettigen, verzweigten oder cyclischen, gesättigten oder ungesättigten aliphatischen KW-Rest mit 1 bis 6 C-Atomen, der noch durch C₆-C₁₂-Aryl substituiert sein kann, oder einen Phenyl- oder Naphthylrest oder einen ein- bis dreifach substituierten Phenyl- oder Naphthylrest, wobei die Substituenten aus der Reihe C₁-C₁₂-Alkyl, C₁-C₁₂-Alkoxy, Sulfo, Hydroxy, Amino, C₁-C₁₂-Alkylamino, Di-(C₁-C₁₂-alkyl)amino, C₂-C₅-Acylamido, Acetoacetamido, Aminocarbonyl, C₁-C₄-Alkylamino-carbonyl und Di-(C₁-C₄-alkyl)-aminocarbonyl ausgewählt sind,
Z⁴ einen Acylrest einer monofunktionellen Harzsäure vom Kolophoniumtyp,
Z⁵ eine Gruppe der Formel -SO₃M, in der M ein Kation bedeutet, oder einen Acylrest der Formel -CO-B-COOM, in der B die definierte Bedeutung hat und M für ein Kation steht, und
Z⁶ einen Acylrest der Formel -CO-B-COO-(X-O)ₘ-Y-R³, in der B und X die genannten Bedeutungen haben, Y eine direkte Bindung oder eine Gruppe der Formel CO bedeutet, m im Falle Y = CO eine Zahl von 1 bis 150 und im Falle Y = direkte Bindung eine Zahl von 0 bis 150 und R³ ein gesättigter oder ungesättigter aliphatischer, aromatischer oder araliphatischer C₃-C₂₄-Kohlenwasserstoffrest sind,
bedeuten, wobei mindestens ein Rest Z ein Rest aus der Gruppe Z², Z⁴ und Z⁶ ist,
stehen.

2. Verbindung nach Anspruch 1, dadurch gekennzeichnet, daß
A für C₂-C₁₂-Alkylen, C₆-C₁₂-Arylen, C₇-C₁₄-Alkyl-arylen, C₅-C₇-Cycloalkylen oder C₂-C₂₄-Alkylen, das ein- oder mehrfach durch eine Gruppe der Formel NR′, worin R′ Wasserstoff, C₁-C₄-Alkyl oder eine Gruppe der Formel -(X-O)ₙ- mit einer wie im Anspruch 1 definierten freien Valenz ist, unterbrochen ist, oder für eine Kombination der aufgeführten divalenten Gruppen und
B für geradkettiges C₁-C₈-Alkylen, einen Alkylenrest einer Dicarbonsäure auf Basis dimerisierter C₁₁-C₂₄-Fettsäuren, Cyclohexylen, C₆-C₁₂-Arylen oder eine Gruppe der Formel -CH=CH- oder -CH₂CH(SO₃M)-, worin M ein Kation ist, stehen.

3. Verbindung nach Anspruch 1 oder 2, dadurch gekennzeichnet, daß
A für C₂-C₆-Alkylen, für eine Gruppe der Formel -C₆H₁₀-(C₁-C₄-Alkylen)-C₆H₁₀- oder der Formel -(CₓH₂ₓ-NR′)ₚ-CₓH₂ₓ- ,
in der x 2 bis 4 und p 1 bis 5 sind und R′ Wasserstoff, C₁-C₄-Alkyl oder eine Gruppe der Formel -(X-O)ₙ- mit einer wie in Anspruch 1 definierten freien Valenz bedeutet,
X zu 80 bis 100 % für Gruppen der Formeln -CH₂CH₂-,
B für C₁-C₆-Alkylen, einen Alkylenrest einer Dicarbonsäure auf Basis dimerisierter ungesättigter C₁₆-C₁₈-Fettsäuren, 1,2-, 1,3- oder 1,4-Phenylen, eine Gruppe der Formel -CH=CH- oder -CH₂CH(SO₃M)-, worin M ein Kation ist, und
n für 1 bis 20
stehen.

4. Verbindung nach einem oder mehreren der Ansprüche 1 bis 3, dadurch gekennzeichnet, daß
Z² ein Alkylrest einer Fettsäure mit 12 bis 18 C-Atomen ist.

5. Verbindung nach einem oder mehreren der Ansprüche 1 bis 4, dadurch gekennzeichnet, daß
Z³ Phenyl, Naphthyl, ein ein- bis dreifach substituierter Phenyl- oder Naphthylrest ist, wobei die Substituenten aus der Reihe C₁-C₄-Alkyl, C₁-C₄-Alkoxy, Hydroxy, Amino, Acetamido oder Acetoacetamido ausgewählt sind.

6. Verbindung nach einem oder mehreren der Ansprüche 1 bis 5, dadurch gekennzeichnet, daß
Z⁵ einen Acylrest der Formel -CO-B-COOM, in der B eine Gruppe der Formel -CH₂-CH(SO₃M)- oder -C₆H₄- und M ein Kation ist, bedeutet.

7. Verbindung nach einem oder mehreren der Ansprüche 1 bis 6, dadurch gekennzeichnet, daß
Z⁶ einen Acylrest der Formel -CO-B-COO-(X-O)ₘ-Y-R³ , in der B und X die genannten Bedeutungen haben, Y eine direkte Bindung oder eine Gruppe der Formel CO bedeutet, m im Falle Y = CO eine Zahl von 1 bis 50 und im Falle Y = direkte Bindung eine Zahl von 0 bis 50 und Y-R³ ein Alkylrest einer Fettsäure mit 8 bis 20 C-Atomen, ein C₈-C₂₀-Alkylrest eines Fettalkohols, ein Acylrest einer monofunktionellen Harzsäure, Phenyl, Naphthyl, C₇-C₂₀-Alkylphenyl oder C₁₁-C₂₄-Alkylnaphthyl sind,
bedeutet.

8. Verfahren zur Herstellung einer nach einem oder mehreren der Ansprüche 1 bis 7 definierten Verbindung mit der Struktur eines modifizierten Blockpolymerisates (I), dadurch gekennzeichnet, daß man Diamin-oxalkylate der Formel (Ia), in der die freien Valenzen an den Polyglykoletherketten durch Z¹ = Wasserstoff abgesättigt sind, teilweise oder ganz mit Carbonsäuren der Formel H-Z oder Gemischen daraus, wobei Z eine oder mehrere der in Anspruch 1 genannten Bedeutungen Z² bis Z⁶ und mindestens eine davon die Bedeutung Z², Z⁴ oder Z⁶ hat, in einer oder mehreren Reaktionsstufen unter Veresterung umsetzt.

9. Verfahren zur Herstellung einer nach einem oder mehreren der Ansprüche 1 bis 7 definierten Verbindung mit der Struktur eines modifizierten Blockpolymerisates (I), dadurch gekennzeichnet, daß man zwei oder mehrere Diaminoxalkylate der Formel (Ia), in der die freien Valenzen an den Polyglykoletherketten durch Z¹ = Wasserstoff abgesättigt sind, in einer ersten Reaktionsstufe durch Veresterung mit einer oder mehreren Dicarbonsäuren der Formel HOOC-B-COOH, worin B die im Anspruch 1 genannte Bedeutung hat, im Molverhältnis von 2:1 bis 10:9 miteinander verknüpft.

10. Verfahren zur Herstellung einer nach einem oder mehreren der Ansprüche 1 bis 7 definierten Verbindung mit der Struktur eines modifizierten Blockpolymersates (I), dadurch gekennzeichnet, daß man zwei oder mehrere Diaminoxalkylate der Formel (Ia), in der die freien Valenzen an den Polyglykoletherketten schon zum Teil mit Monocarbonsäuren des in Anspruch 8 definierten Typs der Formel H-Z verestert sind, in einer zweiten Reaktionsstufe mit einer oder mehreren Dicarbonsäuren der Formel HOOC-B-COOH, worin B die im Anspruch 1 genannte Bedeutung hat, im Molverhältnis von 2:1 bis 10:9 miteinander verknüpft.

11. Verfahren nach einem oder mehreren der Ansprüche 8 bis 10, dadurch gekennzeichnet, daß man zur Einführung von anionischen Resten in die Verbindung mit der Struktur eines modifizierten Blockpolymerisates (I) die Diamin-oxalkylate der Formel (Ia) oder solche teilweise mit Monocarbonsäuren der Formel H-Z veresterte Diamin-oxalkylate, sofern letztere noch freie Hydroxygruppen aufweisen, mit den Sulfato-Rest lieferenden Verbindungen umsetzt.

12. Verfahren nach einem oder mehreren der Ansprüche 8 bis 10, dadurch gekennzeichnet, daß man zur Einführung von anionischen Resten in die Verbindung mit der Struktur eines modifizierten Blockpolymerisates (I) die Diamin-oxalkylate der Formel (Ia) oder solche teilweise mit Monocarbonsäuren der Formel H-Z veresterte Diamin-oxalkylate, sofern letztere noch freie Hydroxygruppen aufweisen, mit einem Überschuß an Dicarbonsäuren der Formel HOOC-B-COOH oder deren Anhydride zu den entsprechenden Halbestern umsetzt.

13. Verfahren nach Anspruch 12, dadurch gekennzeichnet, daß man im Falle von in die Diamin-oxalkylate der Formel (Ia) oder in solche teilweise mit Monocarbonsäuren der Formel H-Z veresterte Diamin-oxalkylate als anionischen Rest eingeführten Maleinsäurehalbestergruppen die letzteren nachträglich durch Umsetzung mit Sulfiten oder Hydrogensulfiten in die entsprechenden Sulfobernsteinsäurehalbester überführt.

14. Verwendung einer nach einem oder mehreren der Ansprüche 1 bis 7 definierten Verbindung mit der Struktur eines modifizierten Blockpolymerisates (I) als oberflächenaktives Mittel.

15. Verwendung nach Anspruch 14 als Kupplungshilfsmittel, Emulgator, Dispergiermittel, Präparationsmittel für Feststoffdispersionen, insbesondere Farbmitteldispersionen, Korrosionsschutz- und Metallbearbeitungsmittel, sowie als Netz- und Färbereihilfsmittel.

16. Verwendung nach Anspruch 15 als Kupplungshilfsmittel und Präparationsmittel für die Herstellung von Azopigmenten und Azofarbstoffen.

17. Verwendung nach Anspruch 15 zur Herstellung von gut fließbaren Pigmentdispersionen für den wäßrigen Druckfarbensektor.

18. Verwendung nach Anspruch 15 als Emulgiermittel bei der Herstellung von Flüssigemulsionen, bevorzugt Mineralöl-und Fettkörperemulsionen.

19. Verwendung nach Anspruch 15 als Emulgier- und Lösevermittler bei der Herstellung von Reinigungsverstärkern und Carrieremulsionen.

## Claims

1. A compound having the structure of a modified block polymer (I), built up, corresponding to the formula, from
a) 1 to 10 trivalent or tetravalent block oxyalkylate units based on diamines or polyamines (aminoxyalkylates) having the formula (Ia) 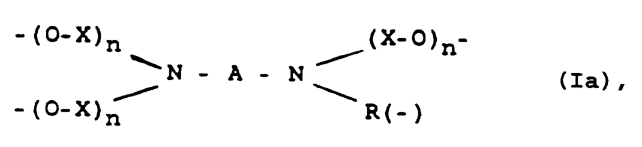
b) monovalent acid radicals symbolized by the formula (Ib)
-Z (Ib)
and, when two or more formula units (Ia) are present, additionally from
c) divalent groups of the formula (Ic)
- CO - B - CO - (Ic),
where each of the free valences indicated in the formula units (Ia) is defined in a manner such that it is bonded, independently of one another, directly to one formula unit (Ib) in each case or to a valence of the formula unit (Ic), and where, in the formula units (Ia) to (Ic),
A represents a straight-chain, branched or cyclic aliphatic radical, an aromatic or araliphatic radical having 1 to 300 carbon atoms in each case,
X represents identical or different groups of the formulae -CH₂CH₂- and -CH₂CH(CH₃)-,
n represents identical or different numbers from 1 to 100,
R represents a C₁-C₃-alkyl group or a divalent group of the formula -(X-O)ₙ- with one free valence as defined above and indicated by (-),
B represents a straight-chain, branched or cyclic aliphatic radical, an aromatic or araliphatic radical having 1 to 60 carbon atoms in each case,
Z represents identical or different radicals Z¹ to Z⁶, in which
Z¹ denotes hydrogen,
Z² denotes an acyl radical of the formula R¹-CO- in which R¹ denotes a straight-chain, saturated or unsaturated C₇-C₂₁-aliphatic hydrocarbon radical which, in addition, may be substituted by one or two hydroxyl groups or by one C₆-C₁₂-aryl or C₆-C₁₂-hydroxyaryl radical,
Z³ denotes an acyl radical of the formula R²-CO- in which R² denotes a straight-chain, branched or cyclic, saturated or unsaturated aliphatic hydrocarbon radical having 1 to 6 carbon atoms which, in addition, may be substituted by C₆-C₁₂-aryl, or denotes a phenyl or naphthyl radical or a monosubstituted to trisubstituted phenyl or naphthyl radical, the substituents being selected from the series comprising C₁-C₁₂-alkyl, C₁-C₁₂-alkoxy, sulfo, hydroxyl, amino, C₁-C₁₂-alkylamino, di-(C₁-C₁₂-alkyl)amino, C₂-C₅-acylamido, acetoacetamido, aminocarbonyl, C₁-C₄-alkylaminocarbonyl and di-(C₁-C₄-alkyl)aminocarbonyl,
Z⁴ denotes an acyl radical of a monofunctional resin acid of the colophonium type,
Z⁵ denotes a group of the formula -SO₃M in which M denotes a cation, or an acyl radical of the formula -CO-B-COOM in which B has the defined meaning and M represents a cation, and
Z⁶ denotes an acyl radical of the formula -CO-B-COO-(X-O)ₘ-Y-R³, in which B and X have the meanings mentioned, Y is a direct bond or a group of the formula CO, m is a number from 1 to 150 in the case where Y = CO and a number from 0 to 150 in the case where Y = a direct bond, and R³ is a saturated or unsaturated aliphatic, aromatic or araliphatic C₃-C₂₄-hydrocarbon radical,
where at least one radical Z represents a radical from the group comprising Z², Z⁴ and Z⁶.

2. A compound as claimed in claim 1, wherein
A represents C₂-C₁₂-alkylene, C₆-C₁₂-arylene, C₇-C₁₄-alkylarylene, C₅-C₇-cycloalkylene or C₂-C₂₄-alkylene which is interrupted one or more times by a group of the formula NR' in which R' is hydrogen, C₁-C₄-alkyl or a group of the formula -(X-O)ₙ- having one free valence as defined in claim 1, or represents a combination of the divalent groups listed, and
B represents straight-chain C₁-C₈-alkylene, an alkylene radical of a dicarboxylic acid based on dimerized C₁₁-C₂₄-fatty acids, cyclohexylene, C₆-C₁₂-arylene or a group of the formula -CH=CH- or -CH₂CH(SO₃M)- in which M is a cation.

3. A compound as claimed in claim 1 or 2, wherein
A represents C₂-C₆-alkylene, a group of the formula -C₆H₁₀-(C₁-C₄-alkylene)-C₆H₁₀- or of the formula -(CₓH₂ₓ-NR')p-CₓH₂ₓ-,
in which x is 2 to 4 and p is 1 to 5, and R' denotes hydrogen, C₁-C₄-alkyl or a group of the formula -(X-O)ₙ- having one free valence as defined in claim 1,
80 to 100% of the X represent groups of the formula -CH₂CH₂-,
B represents C₁-C₆-alkylene, an alkylene radical of a dicarboxylic acid based on dimerized unsaturated C₁₆-C₁₈-fatty acids, 1,2-, 1,3- or 1,4-phenylene, or a group of the formula -CH=CH- or -CH₂CH(SO₃M)- in which M is a cation, and
n represents 1 to 20.

4. A compound as claimed in one or more of claims 1 to 3, wherein
Z² is an alkyl radical of a fatty acid having 12 to 18 carbon atoms.

5. A compound as claimed in one or more of claims 1 to 4, wherein
Z³ is phenyl, naphthyl, or a monosubstituted to trisubstituted phenyl or naphthyl radical, the substituents being selected from the series comprising C₁-C₄-alkyl, C₁-C₄-alkoxy, hydroxyl, amino, acetamido or acetoacetamido.

6. A compound as claimed in one or more of claims 1 to 5, wherein
Z⁵ denotes an acyl radical of the formula -CO-B-COOM in which B is a group of the formula -CH₂-CH(SO₃M)- or -C₆H₄-, and M is a cation.

7. A compound as claimed in one or more of claims 1 to 6, wherein
Z⁶ denotes an acyl radical of the formula -CO-B-COO-(X-O)ₘ-Y-R³, in which B and X have the meanings mentioned, Y is a direct bond or a group of the formula CO, m is a number from 1 to 50 in the case where Y = CO and a number from 0 to 50 in the case where Y = a direct bond, and Y-R³ is an alkyl radical of a fatty acid having 8 to 20 carbon atoms, a C₈-C₂₀-alkyl radical of a fatty alcohol, an acyl radical of a monofunctional resin acid, phenyl, naphthyl, C₇-C₂₀-alkylphenyl or C₁₁-C₂₄-alkylnaphthyl.

8. A process for the preparation of a compound as defined in one or more of claims 1 to 7 and having the structure of a modified block polymer (I), which comprises reacting diamine-oxyalkylates of the formula (Ia) in which the free valences on the polyglycol ether chains are saturated by Z¹ = hydrogen, partially or fully with carboxylic acids of the formula H-Z or mixtures thereof where Z has one or more of the meanings Z² to Z⁶ mentioned in claim 1 and at least one thereof has the meaning Z², Z⁴ or Z⁶, in one or more reaction steps with esterification.

9. A process for the preparation of a compound as defined in one or more of claims 1 to 7 and having the structure of a modified block polymer (I), which comprises linking two or more diamine-oxyalkylates of the formula (Ia) in which the free valences on the polyglycol ether chains are saturated by Z¹ = hydrogen, with one another in a molar ratio from 2:1 to 10:9 in a first reaction step by esterification using one or more dicarboxylic acids of the formula HOOC-B-COOH in which B has the meaning mentioned in claim 1.

10. A process for the preparation of a compound as defined in one or more of claims 1 to 7 and having the structure of a modified block polymer (I), which comprises linking two or more diamine-oxyalkylates of the formula (Ia) in which some of the free valences on the polyglycol ether chains have already been esterified using monocarboxylic acids of the type of the formula H-Z defined in claim 8, with one another in a molar ratio from 2:1 to 10:9 in a second reaction step with one or more dicarboxylic acids of the formula HOOC-B-COOH in which B has the meaning mentioned in claim 1.

11. The process as claimed in one or more of claims 8 to 10, wherein, in order to introduce anionic radicals into the compound having the structure of a modified block polymer (I), the diamine-oxyalkylates of the formula (Ia) or diamine-oxyalkylates which have been partially esterified using monocarboxylic acids of the formula H-Z, so long as the latter diamine-oxyalkylates still contain free hydroxyl groups, are reacted with compounds which supply the sulfato radical.

12. The process as claimed in one or more of claims 8 to 10, wherein, in order to introduce anionic radicals into the compound having the structure of a modified block polymer (I), the diamine-oxyalkylates of the formula (Ia) or diamine-oxyalkylates which have been partially esterified using monocarboxylic acids of the formula H-Z, so long as the latter diamine-oxyalkylates still contain free hydroxyl groups, are reacted with an excess of dicarboxylic acids of the formula
HOOC-B-COOH or the anhydrides thereof to form the corresponding monoesters.

13. The process as claimed in claim 12, wherein, in the case of maleic acid monoester groups introduced as anionic radical into the diamine-oxyalkylates of the formula (Ia) or into diamine-oxyalkylates which have been partially esterified using monocarboxylic acids of the formula H-Z, the maleic acid monoester groups are subsequently converted into the corresponding sulfosuccinic acid monoesters by reaction with sulfites or bisulfites.

14. The use of a compound as defined in one or more of claims 1 to 7 and having the structure of a modified block polymer (I) as a surface-active agent.

15. The use as claimed in claim 14 as a coupling auxiliary, emulsifier, dispersant, formulation agent for solid dispersions, in particular colorant dispersions, corrosion-protection agents and metalworking agents, and as wetting agents and dyeing auxiliaries.

16. The use as claimed in claim 15 as a coupling agent and formulation agent for the preparation of azo pigments and azo dyes.

17. The use as claimed in claim 15 for the preparation of pigment dispersions which have good flow properties, for the agueous printing inks sector.

18. The use as claimed in claim 15 as an emulsifier in the preparation of liquid emulsions, preferably mineral oil emulsions and fatty component emulsions.

19. The use as claimed in claim 15 as an emulsifier and solubilizer in the preparation of cleaning boosters and carrier emulsions.

## Revendications

1. Composé avec la structure d'un polymère séquencé modifié (L) constitué selon la formule à partir
a) de 1 à 10 motifs d'alcoxylate séquencé trivalent ou tétravalent à base de diamines ou polyamines (aminoalcoxylates) de formule générale (Ia) 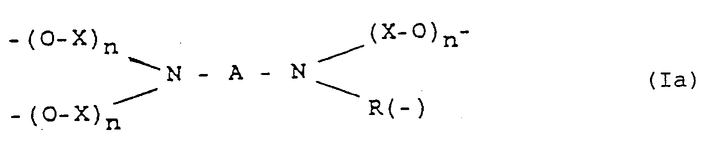
b) de radicaux acides monovalents, symbolisés par la formule générale (Ib)
-Z (Ib)
et, si deux ou plusieurs unités de formule (Ia) sont présentes, de plus,
c) de groupes bivalents de formule générale (Ic)
- CO - B - CO - (Ic)
chacune des valences libres montrées dans les unités de formule (Ia) étant définie de telle façon qu'elle soit liée directement, indépendamment l'une de l'autre, avec chaque fois une unité de formule (Ib) ou une valence de l'unité de formule (Ic) et dans les unités de formule (Ia) à (Ic)
A représentant un radical aliphatique linéaire, ramifié ou cyclique, un radical aromatique ou araliphatique avec chaque fois 1 à 300 atomes de carbone,
X représentant les groupes identiques ou différents de formules -CH₂CH₂- et -CH₂CH(CH₃)-
n représentant les nombres identiques ou différents de 1 à 100,
R représentant un groupe alkyle en C₁-C₃ ou un groupe bivalent de formule -(X-O)ₙ- avec une valence libre telle que définie ci-dessus et indiquée par (-),
B représentant un radical aliphatique linéaire, ramifié ou cyclique, un radical aromatique ou araliphatique avec chaque fois 1 à 60 atomes de carbone, et
Z représentant les radicaux Z¹ à Z⁶ identiques ou différents, où
Z¹ représente l'hydrogène
Z² représente un radical acyle de formule R¹-CO-, dans laquelle R¹ représente un radical KW aliphatique en C₇-C₂₁ linéaire, saturé ou insaturé, qui peut être substitué encore par un ou deux groupes hydroxy ou un radical aryle en C₆-C₁₂ ou hydroxy-aryle en C₆-C₁₂,
Z³ représente un radical acyle de formule R²-CO-, dans laquelle R² représente un radical KW alipriatique linéaire, ramifié ou cyclique insaturé ou saturé, comportant 1 à 6 atomes de carbone, qui peut être substitué encore par un aryle en C₆-C₁₂, ou représente un radical phényle ou naphtyle ou représente un radical phényle ou naphtyle substitué une à trois fois, les substituants étant pris dans la série des alkyle en C₁-C₁₂, alcoxy en C₁-C₁₂, sulfo, hydroxy, amino, alkylamino en C₁-C₁₂, di-(alkyle en C₁-C₁₂)amino, acylamido en C₂-C₅, acétoacétamido, aminocarbonyle, (alkyle en C₁-C₄)aminocarbonyle et di(alkyle en C₁-C₄)aminocarbonyle,
Z⁴ représente un radical acyle d'un acide résinique monofonctionnel de type colophane,
Z⁵ représente un groupe de formule -SO₃M, dans laquelle M signifie un cation, ou un radical acyle de formule -CO-B-COOM dans laquelle B a la signification définie et M représente un cation, et
Z⁶ représente un radical acyle de formule -CO-B-COO-(X-O)ₘ-Y-R³, dans laquelle B et X ont les significations citées, Y signifie une liaison directe ou un groupe de formule CO, m, dans le cas où Y = CO, vaut de 1 à 150, et dans le cas où Y = liaison directe, vaut de 0 à 150 et R³ représente un radical hydrocarboné en C₃-C₂₄ aliphatique, aromatique ou araliphatique, saturé ou insaturé,
au moins un radical Z représentant un radical pris dans le groupe comportant Z², Z⁴ et Z⁶.

2. Composé selon la revendication 1, caractérisé en ce que
A représente les alkylène en C₂-C₁₂, arylène en C₆-C₁₂, alkylarylène en C₇-C₁₄, cycloalkylène en C₅-C₇ ou alkylène en C₂-C₂₄, qui est interrompu une ou plusieurs fois par un groupe de formule NR', où R' représente l'hydrogène, un alkyle en C₁-C₄ ou un groupe de formule -(X-O)ₙ- avec une valence libre comme définie dans la revendication 1, ou correspond à une combinaison des groupes divalents énumérés et
B représente un alkylène en C₁-C₉ linéaire, un radical alkylène d'un acide dicarboxylique à base d'acides gras en C₁₁-C₂₄ dimérisés, cyclohexylène, un arylène en C₆-C₁₂ ou un groupe de formule -CH=CH- ou -CH₂CH(SO₃M)-, où M représente un cation.

3. Composé selon la revendication 1 ou 2, caractérisé en ce que
A correspond à des alkylènes en C₂-C₆, un groupe de formule - C₆H₁₀-(C₁-C₄-alkylène)-C₆H₁₀ - ou de formule - (CₓH₂ₓ-NR')ₚ-CₓH₂ₓ-,
dans laquelle x vaut 2 à 4, et p vaut 1 à 5, et R' représente l'hydrogène, un alkyle en C₁-C₄ ou un groupe de formule -(X-O)ₙ- avec une valence libre comme définie ci-dessus,
X représente 80 à 100 % des groupes de formule -CH₂CH₂-
B représente un alkylène en C₁-C₆, un radical alkylène d'un acide dicarboxylique à base d'un acide gras en C₁₆-C₁₈ insaturé dimérisé, les 1,2-, 1,3- ou 1,4-phénylène, un groupe de formule -CH=CH- ou -CH₂CH(SO₃M)- où M représente un cation, et
n représente 1 à 20.

4. Composé selon une ou plusieurs des revendications 1 à 3, caractérisé en ce que
Z² représente un radical alkyle d'un acide gras comportant de 12 à 18 atomes de carbone.

5. Composé selon une ou plusieurs des revendications 1 à 4, caractérisé en ce que
Z³ représente les phényle, naphtyle, un radical phényle ou naphtyle un à trois fois substitué, les substituants étant pris dans la série des alkyle en C₁-C₄, alcoxy en C₁-C₄, hydroxy, amino, acétamido ou acétoacétamido.

6. Composé selon une ou plusieurs des revendications 1 à 5, caractérisé en ce que
Z⁵ représente un radical acyle de formule -CO-B-COOM, dans laquelle B représente un groupe de formule -CH₂-CH(SO₃M)- ou -C₆H₄- et M représente un cation.

7. Composé selon une ou plusieurs des revendications 1 à 6, caractérisé en ce que
Z⁶ représente un radical acyle de formule -CO-B-COO-(X-O)ₘ-Y-R³, dans laquelle B et X ont les significations données, Y représente une liaison directe ou un groupe de formule CO, m dans le cas où Y = CO représente un nombre de 1 à 50 et dans le cas Y = liaison directe un nombre de 0 à 50 et Y-R³ représente un radical alkyle d'un acide gras comportant de 8 à 20 atomes de carbone, un radical alkyle en C₈-C₂₀ d'un alcool gras, un radical acyle d'un acide résinique monofonctionnel, phényle, naphtyle, alkylphényle en C₇-C₂₀ ou alkyle naphtyle en C₁₁-C₂₄.

8. Procédé pour la préparation d'un composé défini selon une ou plusieurs des revendications 1 à 7 ayant la structure d'un polymère séquencé modifié (I) caractérisé en ce qu'on fait réagir des diaminoalcoxylates de formule (Ia), dans laquelle les valences libres dans les chaînes d'éthers de polyglycols sont saturées par Z¹ = hydrogène, en estérifiant partiellement ou entièrement avec des acides carboxyliques de formule H-Z ou leurs mélanges, Z ayant une ou plusieurs des significations Z² à Z⁶ citées dans la revendication 1 et au moins l'une d'entre elles ayant la signification de Z², Z⁴ ou Z⁶, en une ou plusieurs étapes réactionnelles.

9. Procédé pour la préparation d'un composé défini selon une ou plusieurs des revendications 1 à 7, ayant la structure d'un polymère séquencé modifié (I), caractérisé en ce qu'on lie entre eux deux ou plusieurs diaminoalcoxylates de formule (Ia), dans laquelle les valences libres dans les chaînes d'éthers de polyglycoles sont saturées par Z¹ = hydrogène, dans une première étape réactionnelle par estérification avec un ou plusieurs acides dicarboxyliques de formule HOOC-B-COOH où B a la signification donnée dans la revendication 1, dans un rapport molaire de 2:1 à 10:9.

10. Procédé pour la préparation d'un composé défini selon une ou plusieurs des revendications 1 à 7, ayant la structure d'un polymère séquencé modifié (I), caractérisé en ce qu'on lie entre eux deux ou plusieurs diaminoalcoxylates de formule (Ia), dans laquelle les valences libres dans les chaînes d' éthers de polyglycoles sont déjà estérifiées en partie par des acides monocarboxyliques de formule H-Z de type défini dans la revendication 8, dans une deuxième étape réactionnelle avec un ou plusieurs acides dicarboxyliques de formule HOOC-B-COOH où B a la signification donnée dans la revendication 1, dans un rapport molaire de 2:1 à 10:9.

11. Procédé selon une ou plusieurs des revendications 8 à 10, caractérisé en ce que, pour l'introduction des radicaux anioniques dans le composé ayant la structure d'un polymère séquencé modifié (I), on fait réagir des diaminoalcoxylates de formule (Ia) ou de tels diaminalcoxylates partiellement estérifiés par des acides monocarboxyliques de formule H-Z, dans la mesure où ces derniers présentent des groupes hydroxy encore libres, avec des composés donneurs du radical sulfato.

12. Procédé selon une ou plusieurs des revendications 8 à 10, caractérisé en ce que, pour l'introduction des radicaux anioniques dans le composé ayant la structure d'un polymère séquencé modifié (I), on fait réagir des diaminoalcoxylates de formule (Ia) ou de tels diaminoalcoxylates partiellement estérifiés par des acides monocarboxyliques de formule H-Z, dans la mesure où ces derniers présentent des groupes hydroxy encore libres, avec un excès en acides dicarboxyliques de formule HOOC-B-COOH ou leurs anhydrides pour obtenir des hémi-esters correspondants.

13. Procédé selon la revendication 12, caractérisé en ce que, dans le cas de groupes hémi-esters de l'acide maléique introduits en tant que radical anionique dans les diaminoalcoxylates de formule (Ia) ou dans de tels diaminoalcoxylates partiellement estérifiés par des acides monocarboxyliques de formule H-Z, on transforme ultérieurement ces groupes hémi-esters de l'acide maléique par réaction avec des sulfites ou bisulfites en les hémi-esters de l'acide sulfosuccinique correspondants.

14. Utilisation d'un des composés définis dans l'une ou plusieurs des revendications 1 à 7, ayant la structure d'un polymère séquencé modifié (I) en tant qu'agents de surface.

15. Utilisation selon la revendication 14 en tant qu'agents auxiliaires de copulation, émulsifiants, agents de dispersion, agents de préparation pour les dispersions de matières solides, plus particulièrement de dispersions de colorants, agents de protection contre la corrosion et agents pour le traitement de métaux, ainsi qu'agents de mouillage et de teinture.

16. Utilisation selon la revendication 15, en tant qu'agents de copulation et agents de préparation pour la préparation de pigments azoïques et de colorants azoïques.

17. Utilisation selon la revendication 15 pour la préparation de dispersions pigmentaires fluides pour le secteur des encres d'imprimerie aqueuses.

18. Utilisation selon la revendication 15, en tant qu'émulsifiants pour la préparation d'émulsions liquides, de préférence des émulsions d'huiles minérales et de corps gras.

19. Utilisation selon la revendication 15, en tant qu'émulsifiants et agents solubilisants dans la préparation d'agents renforçant la détergence et d'émulsions-véhicules.
